# EUROPÄISCHE PATENTSCHRIFT

(11) **EP 0 428 022 B1**
(45) Veröffentlichungstag und Bekanntmachung des Hinweises auf die Patenterteilung: **19.01.1994**
(21) Anmeldenummer: 90120996.5
(22) Anmeldetag: 02.11.1990
(51) Int. Cl.: C07C 209/86, C07C 275/06, C07C 273/18, B01D 61/36

(54) **Verfahren zur Abtrennung von Wasser aus einem Wasser, CO2 und Amine enthaltenden Gemisch**
Process for eliminating water from a mixture containing water, carbondioxide and amine
Procédé pour éliminer de l'eau d'un mélange contenant de l'eau, du bioxyde de carbone et de l'amine

(30) Priorität: 14.11.1989 DE 3937796
(43) Veröffentlichungstag der Anmeldung: 22.05.1991
(73) Patentinhaber: BASF Aktiengesellschaft, 67063 Ludwigshafen (DE)
(72) Erfinder: Spiske, Luise, Dr., W-6104 Seeheim-Jugenheim (DE); Luetzow, Dietrich, Dr., W-6703 Limburgerhof (DE); Herion, Christof, Dr., W-6802 Ladenburg (DE); Kuessner, Klaus, Dr., W-6710 Frankenthal (DE); Hefner, Werner, Dr., W-6840 Lampertheim (DE)

(56) Entgegenhaltungen:
- DE-A- 2 602 769
- DE-A- 3 824 488
- DE-B- 1 768 256
- CHEMICAL ABSTRACTS, Band 97, Nr. 22, 29. November 1982, Columbus, Ohio, USA S. YAMADA et al. "Separation of some aqueous amine solutions by pervapor- ation through poly(4-vinyl- pyridine)membrane" Seite 53, Spalte 1, Zusammen- fassung-Nr. 183 550z

## Beschreibung

Die vorliegende Erfindung betrifft ein Verfahren zur Abtrennung von Wasser aus einem Wasser, CO₂ und ein oder mehrere Amine enthaltenden Gemisch durch Pervaporation.

Bei chemischen Verfahren, bei den, neben dem Wertprodukt als weiteres Reaktionsprodukt Wasser gebildet wird, ist es bei der Rückführung von nicht umgesetztem Ausgangsprodukt in den Reaktor in der Regel erforderlich, aus dem Kreislaufstrom einen Seitenstrom abzuzweigen, um den Wassergehalt im Kreislaufstrom nicht zu stark ansteigen zu lassen, da hierdurch der Umsatz zum Wertprodukt herabgesetzt werden kann. Der Seitenstrom enthält in der Regel neben dem Wasser noch Ausgangsstoffe, die vom Wasser getrennt und in den Reaktor zurückgeführt werden. Eine solche Trennung kann beispielsweise durch Destillation erfolgen.

Eine solche destillative Auftrennung eines Seitenstromes ist jedoch problematisch, wenn ein solcher Seitenstrom neben dem Wasser Amine als zurückzugewinnende Ausgangsstoffe und CO₂ enthält. Ein solcher Amin, Wasser und CO₂ enthaltender Seitenstrom wird beispielsweise bei der Herstellung von Dialkylharnstoffen durch Umsetzung von überschüssigen Monoalkylaminen mit CO₂ (z.B. DE-PS 937 586 und DE-PS 1 768 256) erhalten. Bei der destillativen Trennung dieser Seitenströme werden CO₂ und die Amine als Kopfprodukt erhalten. Beim Abkühlen des Kopfproduktes besteht jedoch die Gefahr der Ablangerung von festem Carbamat, wodurch erhebliche verfahrenstechnische Probleme entstehen können.

Aus der DE-PS 1 768 256 ist es bekannt, zur Vermeidung der Carbamat-Bildung bei der Destillation den Amin, CO₂ und Reaktionswasser enthaltenden Strom in der Destillationskolonne mit Natronlauge zu behandeln, wodurch das CO₂ als Natriumcarbonat gebunden wird, das als wäßrige Lösung aus der Destillationskolonne ausgeschleust wird. Es ist ein Nachteil dieses Verfahrens, daß erhebliche Natronlauge-Mengen zum Binden des CO₂ benötigt werden und daß die Beseitigung oder Weiterverwendung der erhaltenen wäßrigen Natriumcarbonatlösung problematisch sein kann.

Es bestand daher Bedarf nach einem Verfahren zur Abtrennung von Wasser aus einem Wasser, CO₂ und Amin enthaltenden Gemisch, welches die Nachteile der bekannten Verfahren vermeidet oder vermindert.

Es wurde nun ein vorteilhaftes Verfahren gefunden zur Abtrennung von Wasser aus einem Wasser, CO₂ und ein oder mehrere Amine enthaltenden Gemisch, welches dadurch gekennzeichnet ist, daß das Wasser aus dem Gemisch durch Pervaporation abgetrennt wird, indem das Gemisch mit einer Seite einer Membran in Berührung gebracht wird und das Wasser enthaltende Permeat als Dampf von der anderen Seite der Membran entfernt wird.

Mit dem neuen Verfahren läßt sich in einfacher Weise Wasser aus einem Wasser, CO₂ und Amine enthaltenden Gemisch abtrennen. Eine Bindung des CO₂ durch Zugabe von Alkalilauge ist bei der Abtrennung des Wassers durch das erfindungsgemäße Verfahren nicht erforderlich.

Für das erfindungsgemäße Verfahren werden im allgemeinen wasserselektive Membranen verwendet. Wegen des Amin-Gehaltes des aufzutrennenden Gemisches werden zweckmäßig basenbeständige Membranen eingesetzt.

Geeignete Membranen sind z.B. porenfreie Membranen auf der Basis von Polyvinylalkohol.

Geeignete Vorrichtungen für die Trennung von Gemischen nach dem erfindungsgemäßen Verfahren durch Pervaporation sind z.B. in der Weise aufgebaut, daß sie eine Rohgemischkammer für das aufzutrennende Gemisch, eine Permeatkammer für das abgetrennte Permeat und eine zwischen diesen angeordnete Membran enthalten sowie mit zumindest einem mit der Rohgemischkammer verbundenen Zulaufkanal und zumindest einem mit der Permeatkammer verbundenen Ablaufkanal versehen sind (vgl. z.B. DE-A- 3 529 175). Die Rohgemischkammer enthält zweckmäßig noch einen Ablaufkanal für den Teil des Gemischs (Retentat), der von der Membran zurückgehalten wird.

Die erfindungsgemäße Pervaporation wird zweckmäßig in der Weise durchgeführt, daß die Konzentration des Wassers im Permeat höher ist als die Konzentration des Wassers im eingesetzten Gemisch und die Amin-Konzentration, bezogen auf als freies Amin und als Carbamat vorliegendes Amin, und die CO₂-Konzentration, bezogen auf als CO₂ und als Carbamat vorliegendes CO₂, im Permeat niedriger sind als im eingesetzten Gemisch.

Das dampfförmig auf der Permeatseite der Membran erhaltene Permeat kann als solches, d.h. dampfförmig, weitergeleitet werden. Es kann jedoch auch vorteilhaft sein, das erhaltene dampfförmige Permeat anschließend zu kondensieren und flüssig weiterzuleiten. Die Kondensation kann in einer oder mehreren nachgeschalteten Kondensationsstufen erfolgen. Bei der Anwendung von mehreren nachgeschalteten Kondensationsstufen kann es vorteilhaft sein, in den einzelnen Kondensationsstufen unterschiedliche Drücke und/oder Temperaturen anzuwenden. Durch Variation der Verfahrens- bedingungen wie Druck und/oder Temperatur ist es möglich, das Permeat zu fraktionieren, so daß Permeatströme und Permeatteilströme unterschiedlicher Zusammensetzung erhalten werden können.

Es kann weiter vorteilhaft sein, das aus einer vorgeschalteten Kondensationsstufe erhaltene, nicht kondensierte Permeat zu komprimieren und dampfförmig weiterzuleiten.

Das erfindungsgemäße Verfahren wird zur Abtrennung von Wasser aus einem Wasser, CO₂ und ein oder mehrere Amine enthaltenden Gemisch angewandt, wobei die Amine primär, sekundär oder tertiär sein können. Vorzugsweise werden sekundäre Amine und insbesondere primäre Amine enthaltende Gemische eingesetzt. Zweckmäßig verwendet man als Amine Alkylamine mit 1 bis 8 C-Atomen, vorzugsweise 1 bis 6 C-Atomen, insbesondere 1 bis 3 C-Atomen, in den Alkylgruppen, wobei bei den sekundären und tertiären Aminen die Alkylgruppen gleich oder verschieden sein können. Geeignete Amine sind z.B. Methylamin, Ethylamin, Propylamin, Hexylamin, Dimethylamin, Diethylamin, Methyl-isopropylamin, Trimethylamin.

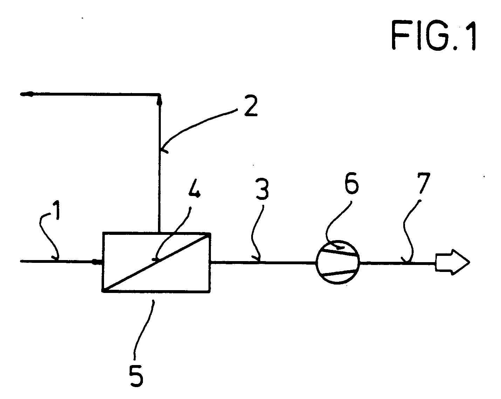

Nachfolgend werden weitere Einzelheiten der Erfindung anhand von Ausführungsbeispielen, deren Verfahrensablauf in den Figuren 1 bis 3 schematisch dargestellt ist, erläutert.

In den Figuren 1 bis 3 bedeuten:
- 5: Vorrichtung zur Trennung des CO₂/H₂O/Amin-Gemisches durch Pervaporation ("Pervaporation")
- 4: die in der Pervaporationsvorrichtung enthaltene Membran
- 1: Zuführung des aufzutrennenden Gemischs
- 2: Ablaufleitung für Retentatstrom
- 3: Ablaufleitung für Permeat

Gemäß Fig. 1 wird der in der Pervaporation 5 erzeugte Permeatdampf mit einer druckgeregelten Vakuumpumpe 6, die einen konstanten Druck aufrechterhält, über Leitung 7 dampfförmig weitergeleitet. Bei sonst konstanten Bedingungen wird die Zusammensetzung des Permeates durch den über die Druckregelung vorgegebenen Permeatdruck bestimmt.

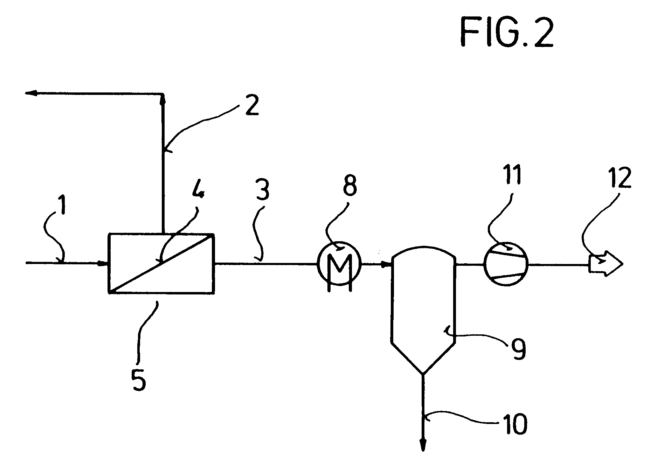

Gemäß Fig. 2 wird der Permeatdampf über Leitung 3 im Wärmetauscher 8 abgekühlt, das erhaltene Kondensat im Behälter 9 gesammelt und über Leitung 10 abgeführt. Ein geringer nicht kondensierbarer Teilstrom wird mit der druckgeregelten Vakuumpumpe 11 über Leitung 12 abgeführt, wobei der durch die Vakuumpumpe vorgegebene Druck zweckmäßig höher als der Dampfdruck des Kondensats gewählt wird. In der Regel liegt der durch die Vakuumpumpe vorgegebene Druck 1 bis 200 mbar, vorzugsweise 1 bis 100 mbar, insbesondere 1 bis 50 mbar, höher als der Dampfdruck des Kondensats und niedriger als der Dampfdruck des eingesetzten Gemischs. Der Permeatdruck und damit dessen Einfluß auf die Selektivität der Trennung und somit die Permeatzusammensetzung wird bei dieser Verfahrensweise durch den Dampfdruck des Permeates bei der Kondensationstemperatur bestimmt. Die Temperatur des eingesetzten Gemisches liegt dabei im allgemeinen zwischen 20 und 150°C, vorzugsweise zwischen 60 und 100°C, insbesondere zwischen 70 und 90°C.

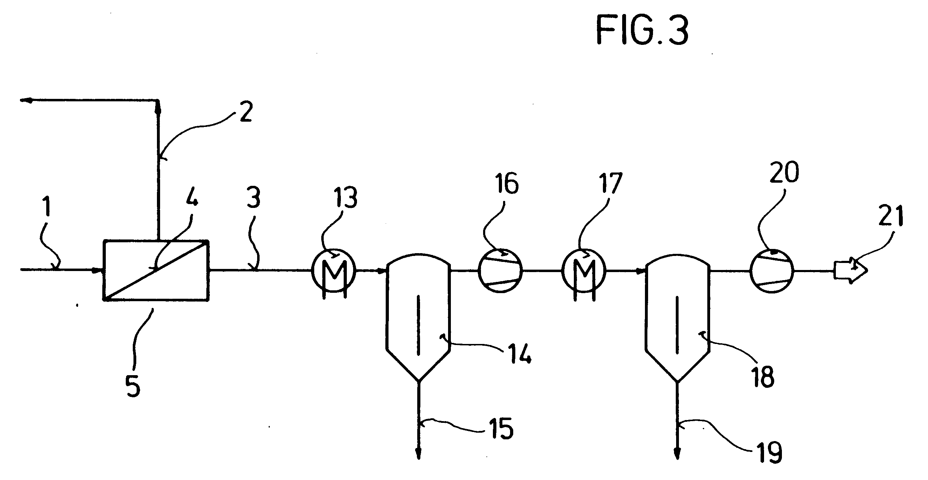

Gemäß Fig. 3 wird der Permeatdampf über Leitung 3 und nach Abkühlung im Wärmetauscher 13 im Behälter 14 teilweise kondensiert, und das Teilkondensat wird über Leitung 15 abgezogen. Der nicht kondensierte Anteil des Permeates wird anschließend im druckgeregelten Kompressor 16 verdichtet und nach Abkühlung im zweiten Wärmetauscher 17 im Behälter 18 kondensiert und über Leitung 19 abgezogen. Der nicht kondensierbare Rest des Permeates wird mit einem weiteren druckgeregelten Kompressor 20 über Leitung 21 weitergeleitet. Diese Art der Permeatabfuhr und Permeat-Behandlung ermöglicht die Aufteilung des Permeats auf mehrere Teilkondensate unterschiedlicher Zusammensetzung, wobei die Aufteilung in bezug auf die Mengen der Teilkondensate und deren Zusammensetzung durch die Wahl der Drücke und der Kondensationstemperaturen in weiten Bereichen variiert werden kann, so daß in einfacher Weise eine Fraktionierung des Permeates durchgeführt werden kann. Bei Anwendung mehrerer Kondensationsstufen werden im allgemeinen 2 bis 5, vorzugsweise 2 bis 3 Kondensationsstufen verwendet.

Alternativ zur Arbeitsweise gemäß Fig. 3 kann das Permeat auch in mehreren Stufen komprimiert und das jeweils verbliebene dampfförmige Permeat weitergeleitet werden. Bei Anwendung mehrerer Kompressionsstufen werden im allgemeinen 2 bis 5, vorzugsweise 2 bis 3 Kompressionsstufen verwendet.

Eine Ausführungsform des erfindungsgemäßen Verfahrens besteht beispielsweise darin, daß man es auf einen Wasser, CO₂ und Monoalkylamine enthaltenden Strom anwendet, den man als Kreislaufstrom erhält bei einem Verfahren zur Herstellung von Dialkylharnstoffen durch Umsetzung von Monoalkylaminen mit CO₂ in einer Reaktionszone bei erhöhter Temperatur und erhöhtem Druck und Abtrennung des nicht umgesetzten Monoalkylamins und CO₂ und des gebildeten Wassers von dem gebildeten Dialkylharnstoff und Rückführung des abgetrennten nicht umgesetzten Monoalkylamins und CO₂ und des nach der Wasserabtrennung gegebenenfalls verbliebenen Wassers im Kreislaufstrom in die Reaktionszone.

Als Ausgangsstoffe für die Herstellung der Dialkylharnstoffe verwendet man Monoalkylamine und CO₂ im allgemeinen in einer Menge von 2 bis 5 mol, vorzugsweise 2 bis 3 mol Monoalkylamin, bezogen auf ein mol CO₂. Bevorzugte Monoalkylamine sind solche mit 1 bis 8, vorzugsweise 1 bis 4 Kohlenstoffatomen, z.B. Methyl-, Ethyl-, Propyl-, Isobutyl-, Hexylamin. Die Umsetzung wird im allgemeinen bei einer Temperatur von 150 bis 220°C, vorzugsweise von 180 bis 200°C und bei einem Druck von 30 bis 160 bar, vorzugsweise 100 bis 150 bar, vorzugsweise kontinuierlich durchgeführt.

Nach der Umsetzung werden die unumgesetzten Ausgangsstoffe vom Dialkylharnstoff in der Regel durch Entspannen und Erhitzen abgetrennt und in einem das Monoalkylamin, CO₂ und Wasser enthaltenden Kreislaufstrom in den Reaktor zurückgeführt. Um die Konzentration des sich durch die Umsetzung bildenden Wassers in dem Kreislaufstrom nicht zu stark ansteigen zu lassen, wird der Kreislaufstrom ganz oder teilweise gemäß dem erfindungsgemäßen Verfahren zur Abtrennung des Wassers einer Pervaporation unterworfen, wobei ein Permeat mit einer höheren Wasser-Konzentration und einer niedrigeren CO₂- und Monoalkylamin-Konzentration als in dem der Pervaporation zugeführten Kreislaufstrom erhalten wird. Entsprechend weist das aus der Pervaporation abgezogene und in den Reaktor zurückgeführte Retentat eine niedrigere Wasser-Konzentration und eine höhere CO₂- und Monoalkylamin-Konzentration als der der Pervaporation zugeführte Kreislaufstrom auf.

Das neben dem Hauptbestandteil Wasser noch in geringerer Konzentration CO₂ und Monoalkylamin enthaltende Permeat wird zweckmäßig zur Wiedergewinnung des Monoalkylamins mit Alkalilauge, vorzugsweise Natronlauge, z.B. wäßrige 10 bis 25 gew.-%ige Natronlauge, behandelt, wobei man stöchiometrische Mengen an Alkalihydroxid, bezogen auf die zu bindende Menge CO₂ anwenden oder mit einem Überschuß, z.B. bis zum 1,1-fachen Überschuß über die stöchiometrische Menge arbeiten kann.

Die Behandlung des Permeats mit der Alkalilauge wird im allgemeinen bei einer Temperatur von 80 bis 130°C, vorzugsweise 90 bis 110°C, und bei einem Druck von 1 bis 3 bar durchgeführt, wobei man vorteilhaft eine Destillationskolonne z.B. in der Weise verwendet, daß man das Permeat in den mittleren Teil der Destillationskolonne einleitet, während die Alkalilauge am Kopf der Kolonne zugeführt wird. Am Kolonnenkopf wird dabei das Monoalkylamin abgezogen, das zweckmäßig in den Reaktor für die Umsetzung zum Dialkylharnstoff zurückgeführt wird. Am Kolonnenboden wird eine wäßrige Carbonatlösung, die das CO₂ in gebundener Form und das Wasser enthält, abgezogen.

Die folgenden Beispiele veranschaulichen die Erfindung.

### Beispiel 1

Aus der Umsetzung von Methylamin mit CO₂ zu Dimethylharnstoff in einem Druckreaktor wird nach der Abtrennung des nicht umgesetzten Methylamins und CO₂ und des gebildeten Wassers von dem gebildeten Dimethylharnstoff ein Kreislaufstrom aus 45 Gew.-% Methylamin (freies Methylamin und als Carbamat gebundenes Methylamin), 28 Gew.-% CO₂ (gelöstes CO₂ und als Carbamat gebundenes CO₂) und 27 Gew.-% Wasser erhalten. Um die Wasserkonzentration in dem in den Druckreaktor zurückgeführten Kreislaufstrom durch das Reaktionswasser nicht zu hoch ansteigen zu lassen, wird der Kreislaufstrom bei 80°C und einem Permeatdruck von 20 mbar unter Verwendung einer Lösungsdiffusions-Membran auf der Basis von Polyvinylalkohol einer Pervaporation unterworfen, wobei Membranen in einer Fläche von 250 m² verwendet werden und ein Permeatstrom von 132 kg/h aus 76 Gew.-% Wasser, 3 Gew.-% CO₂ und 21 Gew.-% Methylamin erhalten wird. Mit dem Permeatstrom werden somit 100 kg/h Wasser aus dem Kreislaufstrom ausgetragen. Das aus der Pervaporation erhaltene Retentat wird als verbleibener Kreislaufstrom in den Druckreaktor zurückgeführt.

Der Permeatstrom wird anschließend zur Rückgewinnung des Methylamins in den mittleren Teil einer Destillationskolonne geleitet, in der am Kopf der Kolonne zum Binden des CO₂ 7,2 kg/h NaOH als wäßrige Natronlauge zugegeben werden. Am Sumpf der Kolonne wird wäßrige Natriumcarbonat-Lösung abgezogen. Am Kopf der Kolonne wird Methylamin abgezogen, das in den Druckreaktor zur Umsetzung zum Dimethylharnstoff zurückgeführt wird.

### Vergleichsversuch

In einem Vergleichsversuch wird die Umsetzung von Methylamin mit CO₂ zu Dimethylharnstoff in einem Druckreaktor gemäß Beispiel 1 durchgeführt, wobei wieder ein Kreislaufstrom aus 45 Gew.-% Methylamin, 28 Gew.-% CO₂ und 27 Gew.-% Wasser erhalten wird.

Um aus dem Kreislaufstrom 100 kg/h wasser abzuziehen, wird aus dem Kreislaufstrom gemäß dem bekannten Verfahren ein Seitenstrom von 370 kg/h abgezogen, der zur Rückgewinnung des Methylamins in den mittleren Teil einer Destillationskolonne geleitet wird, in der am Kopf der Kolonne zum Binden des CO₂ 188 kg/h NaOH als wäßrige Natronlauge zugegeben werden. Am Sumpf der Kolonne wird wäßrige Natriumcarbonat-Lösung und am Kopf der Kolonne das Methylamin abgezogen, das in den Druckreaktor zurückgeführt wird.

Gemäß dem Vergleichsversuch wird mit 188 kg/h NaOH eine um den Faktor 26 höhere NaOH-Menge benötigt als gemäß der erfindungsgemäßen Arbeitsweise in Beispiel 1. Entsprechend fällt bei dem erfindungsgemäßen Verfahren eine wesentlich geringere Menge an wäßriger Natriumcarbonat-Lösung an, so daß eine wesentlich geringere Menge an wäßriger Natriumcarbonat-Lösung aufgearbeitet oder entsorgt werden muß.

### Beispiel 2

Ein Kreislaufstrom der Zusammensetzung gemäß Beispiel 1 (45 Gew.-% Methylamin, 28 Gew.-% CO₂ und 27 Gew.-% Wasser) wird gemäß Figur 1 in der Pervaporationsvorrichtung gemäß Beispiel 1 einer Pervaporation unterworfen, wobei das Permeat dampfförmig über Leitung 7 abgezogen wird. Das erhaltene Permeat weist bei einem vorgegebenen Permeatdruck von 30 mbar eine Zusammensetzung von 74 Gew. -% Wasser, 4 Gew.-% CO₂ und 22 Gew.-% Methylamin auf. Erhöht man den vorgegebenen Permeatdruck auf 100 mbar, so erhält man ein Permeat der Zusammensetzung 58 Gew.-% Wasser, 9 Gew.-% CO₂ und 33 Gew.-% Methylamin.

### Beispiel 3

Ein Kreislaufstrom mit der Zusammensetzung gemäß Beispiel 1 wird gemäß Figur 3 in der Pervaporationsvorrichtung gemäß Beispiel 1 bei 80°C einer Pervaporation unterworfen. Das zunächst dampfförmig erhaltene Permeat durchläuft anschließend gemäß Fig. 3 zwei Kondensationsstufen.

In der ersten Kondensationsstufe wird mit der Vakuumpumpe 16 ein Druck von 50 mbar vorgegeben, wobei bei einer Kondensationstemperatur von 0°C (Fall 1) im Wärmetauscher 13 94 Gew.-% des gesamten Permeats als Kondensatstrom 15 der Zusammensetzung 71 Gew.-% Wasser, 9 Gew.-% CO₂ und 20 Gew.-% Methylamin erhalten werden. Wird im Wärmetauscher 13 eine Kondensationstemperatur von 20°C (Fall 2) eingestellt, so wird bei unverändertem Druck das Permeat zu 87 Gew.-%, bezogen auf das gesamte Permeat, in der ersten Kondensationsstufe als Kondensat der Zusammensetzung 80 Gew.-% Wasser, 6 Gew.-% CO₂ und 14 Gew.-% Methylamin über Leitung 15 erhalten.

In der zweiten Kondensationsstufe wird in beiden Fällen (Fall 1 und Fall 2) im Wärmetauscher 17 eine Kondensationstemperatur von 0°C und ein Druck von jeweils 1 bar eingestellt. Dabei werden jeweils die verbliebenen Permeatanteile über Leitung 19 als Kondensat erhalten. Im Fall 1 beträgt die Kondensatmenge in der zweiten Kondensationsstufe ca. 6 Gew.-% und im Fall 2 ca. 13 Gew.-%, jeweils bezogen auf die ursprüngliche über Leitung 13 erhaltene Permeatmenge, wobei das Kondensat in beiden Fällen zu praktisch 100 % aus Methylamin besteht.

## Patentansprüche

1. Verfahren zur Abtrennung von Wasser aus einem Wasser, CO₂ und ein oder mehrere Amine enthaltenden Gemisch, dadurch gekennzeichnet, daß das Wasser aus dem Gemisch durch Pervaporation abgetrennt wird, indem das Gemisch mit einer Seite einer Membran in Berührung gebracht wird und das Wasser enthaltende Permeat als Dampf von der anderen Seite der Membran entfernt wird.

2. Verfahren nach Anspruch 1, dadurch gekennzeichnet, daß für die Pervaporation eine wasserselektive Membran verwendet wird.

3. Verfahren nach Anspruch 1 und 2, dadurch gekennzeichnet, daß die Membran eine aktive Schicht auf der Basis von Polyvinylalkohol besitzt.

4. Verfahren nach Anspruch 1 bis 3, dadurch gekennzeichnet, daß die Konzentration des Wassers im Permeat höher ist als die Konzentration des Wassers im eingesetzten Gemisch und die Aminkonzentration, bezogen auf als freies Amin und als Carbamat vorliegendes Amin, und die CO₂-Konzentration, bezogen auf als CO₂ und als Carbamat vorliegendes CO₂, im Permeat niedriger sind als im eingesetzten Gemisch.

5. Verfahren nach Anspruch 1 bis 4, dadurch gekennzeichnet, daß das Permeat dampfförmig von der Membran weitergeleitet wird.

6. Verfahren nach Anspruch 1 bis 5, dadurch gekennzeichnet, daß das Permeat kondensiert und flüssig weitergeleitet wird.

7. Verfahren nach Anspruch 1 bis 6, dadurch gekennzeichnet, daß das Permeat in mehreren nachgeschalteten Stufen komprimiert und/oder kondensiert wird, wobei in den einzelnen Kompressions- und/oder Kondensationsstufen unterschiedliche Drücke und/oder Temperaturen angewendet werden.

8. Verfahren gemäß Anspruch 1 bis 7, dadurch gekennzeichnet, daß es auf einen Wasser, CO₂ und Monoalkylamine enthaltenden Strom angewandt wird, der als Kreislaufstrom erhalten wird bei einem Verfahren zur Herstellung von Dialkylharnstoffen durch Umsetzung von Monoalkylaminen mit CO₂ in einer Reaktionszone bei erhöhter Temperatur und erhöhtem Druck und Abtrennung des nicht umgesetzten Monoalkylamins und CO₂ und des gebildeten Wassers von dem gebildeten Dialkylharnstoff und Rückführung des abgetrennten nicht umgesetzten Monoalkylamins und CO₂ und des nach der Wasserabtrennung gegebenenfalls verbliebenen Wassers im Kreislaufstrom in die Reaktionszone.

## Claims

1. A process for removing water from a mixture comprising water, CO₂ and one or more amines, which comprises removing the water from the mixture by pervaporation by bringing the mixture into contact with one side of a membrane and removing the water-containing permeate as a vapor from the other side of the membrane.

2. A process as claimed in claim 1, wherein a water-selective membrane is used.

3. A process as claimed in claim 1 or 2, wherein the membrane has an active layer based on polyvinyl alcohol.

4. A process as claimed in any of claims 1 to 3, wherein the concentration of water in the permeate is higher than the concentration of water in the feed mixture and the amine concentration, based on free amine and carbamate amine, and the CO₂ concentration, including carbamate CO₂, are lower in the permeate than in the feed mixture.

5. A process as claimed in any of claims 1 to 4, wherein the permeate is conducted away from the membrane in vapor form.

6. A process as claimed in any of claims 1 to 5, wherein the permeate is condensed and conducted away in liquid form.

7. A process as claimed in any of claims 1 to 6, wherein the permeate is compressed and/or condensed in a plurality of downstream stages, the individual compression and/or condensation stages being maintained under different pressures and/or temperatures.

8. A process as claimed in any of claims 1 to 7, which is applied to a stream comprising water, CO₂ and a monoalkylamine, obtained as a recycle stream in a process for preparing a dialkylurea by reacting a monoalkylamine with CO₂ in a reaction zone at elevated temperature and pressure and separating the unconverted monoalkylamine and CO₂ and the water formed from the dialkylurea formed and recycling the removed unconverted monoalkylamine and CO₂ and any water remaining after the water separation stage into the reaction zone in the recycle stream.

## Revendications

1. Procédé de séparation d'eau d'un mélange contenant de l'eau, du CO₂ et une ou plusieurs amines, caractérisé en ce que l'on sépare l'eau du mélange par pervaporation, pour autant que l'on mette le mélange en contact avec l'un des côtés d'une membrane et on élimine le produit de la perméation contenant de l'eau sous forme de vapeur de l'autre côté de la membrane.

2. Procédé suivant la revendication 1, caractérisé en ce que l'on utilise une membrane hydrosélective pour la pervaporation.

3. Procédé suivant les revendications 1 et 2, caractérisé en ce que la membrane comporte une couche active à base de poly(alcool vinylique).

4. Procédé suivant les revendications 1 à 3, caractérisé en ce que la concentration de l'eau dans le produit de perméation est supérieure à la concentration de l'eau dans le mélange mis en oeuvre au départ et la concentration en amine, rapportée à l'amine libre et à l'amine présente sous forme de carbamate et la concentration en CO₂, rapportée au CO₂ et au CO₂ présent sous forme de carbamate, dans le produit de perméation, sont inférieures à celles existant dans le mélange mis en oeuvre au départ.

5. Procédé suivant les revendications 1 à 4, caractérisé en ce que l'on enlève le produit de perméation de la membrane à l'état de vapeur.

6. Procédé suivant les revendications 1 à 5, caractérisé en ce que l'on enlève le produit de perméation à l'état condensé et liquide.

7. Procédé suivant les revendications 1 à 6, caractérisé en ce que l'on comprime et/ou condense le produit de perméation dans plusieurs étages subséquents, où on utilise des pressions et/ou des températures différentes dans les étages de compression et/ou de condensation individuels.

8. Procédé suivant les revendications 1 à 7, caractérisé en ce qu'on le met en oeuvre sur un courant contenant de l'eau, du CO₂ et des monoalkylamines, que l'on obtient à titre de courant de recyclage lors de l'exploitation d'un procédé de préparation de dialkylurées par réaction de monoalkylamines avec du CO₂ dans une zone de réaction à haute température et à pression élevée et séparation du CO₂ et de la monoalkylamine non convertis, ainsi que de l'eau formée, de la dialkylurée engendrée et recyclage du CO₂ et de la monoalkylamine non convertis et séparés et de l'eau subsistant éventuellement après la séparation d'eau, dans le courant de recyclage dans la zone de réaction.
